(19) Europäisches Patentamt
European Patent Office
Office européen des brevets

(11) Numéro de publication: **0 222 145 B1**

(12) # FASCICULE DE BREVET EUROPEEN

(45) Date de publication du fascicule du brevet: **23.08.89**

(51) Int. Cl.⁴: **C07C 57/03,** C07C 51/12

(21) Numéro de dépôt: **86113777.6**

(22) Date de dépôt: **04.10.86**

(54) **Procédé pour la préparation d'acides carboxyliques et de leurs sels alcalins.**

(30) Priorité: **08.11.85 CH 4807/85**

(43) Date de publication de la demande: **20.05.87 Bulletin 87/21**

(45) Mention de la délivrance du brevet: **23.08.89 Bulletin 89/34**

(84) Etats contractants désignés: **CH DE FR GB LI NL**

(56) Documents cités:
**EP-A- 0 146 859**
**US-A- 4 189 608**

(73) Titulaire: **FIRMENICH SA, 1, route des Jeunes, CH-1211 Genève 8(CH)**

(72) Inventeur: **Rautenstrauch, Valentin, Dr., 69, chemin de Saules, CH-1233 Bernex(CH)**

(74) Mandataire: **Salvadori, Giuseppe, Dr., c/o Firmenich S.A. Case Postale 239, CH-1211 Genève 8(CH)**

**EP 0 222 145 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

ACTORUM AG

## Description

La présente invention a trait à un procédé nouveau pour la préparation d'acides carboxyliques ou de leurs sels alcalins, laquelle invention repose sur une découverte fortuite de notre part. Nous avons en effet observé que certains alcoolates de métaux alcalins, notamment de potassium, réagissaient, en solution dans un solvant organique inerte, avec de l'oxyde de carbone pour fournir les carboxylates correspondants. Il s'agit schématiquement de la réaction que voici :

$R\text{-}OMe + CO \rightarrow RCO_2Me$
(ME = métal alcalin)

Une telle réaction avait été décrite par Geuther et Frölich [voir Liebigs Ann. Chem. 202, 288 (1880)] qui toutefois avaient limité leurs études à des alcoolates dérivés d'alcools aliphatiques simples, linéaires et saturés, le méthylate et l'éthylate de sodium en particulier. D'autre part, au vu de la méthodologie suivie, les rendements observés étaient bas et le procédé s'est révélé peu aisé dans son application industrielle : l'alcoolate alcalin était en effet fondu et traité avec du CO. Ceci peut expliquer la raison pour laquelle cette réaction n'a pas été reprise par la suite et les travaux de Geuther et Frölich sont tombés dans l'oubli.

Or, il existe un intérêt certain à la préparation d'acides carboxyliques portant une insaturation de type allylique ; de tels composés sont en effet difficilement accessibles au moyen des méthodes de synthèse communément pratiquées.

La présente invention apporte une solution nouvelle et industriellement praticable à ce problème.

Le procédé selon l'invention est destiné à la préparation d'acides carboxyliques et de leurs sels alcalins de formule (I) dans laquelle X définit un hydrogène, ou un atome de métal alcalin, et $R^1$ et $R^2$ identiques ou différents, représentent chacun un reste alkyle linéaire ou ramifié, saturé ou insaturé, lequel procédé est caractérisé en ce qu'on

A. traite un alcoolate de formule (II) dans laquelle Me représente un atome de métal alcalin, en solution dans un solvant organique inerte, dans les conditions de la réaction, avec du CO à une pression comprise entre 35 et 450 bar et à une température comprise entre 120 et 160°C, traite ensuite avec de l'eau le carboxylate alcalin obtenu et acidifie enfin la solution aqueuse résultante pour fournir l'acide désiré, ou

B. traite avec du CO un alcoolate de formule (II) en solution dans un solvant organique inerte dans les conditions de la réaction en présence d'un réactif ligand des cations alcalins, à une pression comprise entre 40 et 80 bar et à une température de 15 à 45°C, traite ensuite avec de l'eau le carboxylate alcalin obtenu et acidifie enfin la solution aqueuse résultante pour fournir l'acide désiré.

Dans la formule (I), $R^1$ et $R^2$ peuvent représenter des radicaux alkyles variés. Ainsi ils peuvent définir aussi bien un alkyle linéaire saturé inférieure $C_1$-$C_6$, qu'un radical alkyle comportant une ou plusieurs insaturations. A titre d'exemple, on peut citer les restes méthyle, éthyle, n-propyle, isopropyle, n-butyle, iso-butyle, tert-butyle, le n-pentyle ou des radicaux insaturés tel le vinyle, le crotonyle ou encore le $-(CH_2)_2-CH=C(CH_3)_2$.

A titre d'exemple, il convient de mentionner en tant que produits de départ les alcoolates dérivés du linalol et du 3-vinyl-1-octène-3-ol, respectivement de formule (III) et (IV).

Les alcoolates sont obtenus aisément par les méthodes usuelles à partir de l'alcool correspondant. A titre d'alcoolate préférentiel on emploie l'alcoolate de potassium, lequel est préparé par traitement de l'alcool avec de l'hydrure de potassium. On opère de préférence dans un solvant organique inerte, par exemple un hydrocarbure aromatique tel le benzène. Dans un tel solvant, l'alcoolate de potassium est généralement soluble et la solution résultante peut directement être traitée avec du CO conformément au procédé de l'invention. Cette opération a lieu comme indiqué plus haut à une pression supérieure à la pression atmosphérique et l'on utilise à cet effet un autoclave usuel en acier inoxydable.

Suivant la variante B. du procédé de l'invention, la réaction a lieu en présence d'un réactif ligand des cations alcalins. Des tels réactifs sont connus sous le nom d'éthers couronne (crown ethers). Il s'agit pour la plupart de produits commerciaux, dont la nature dépend de l'emploi spécifique considéré. A titre de réactif ligand préférentiel pour le cation $K^+$, il s'est révélé que le produit connu sous la dénomination 18-couronne-6 [voir E.V. Dehmlow et S.S. Dehmlow, Phase Transfer Catalysis, Verlag Chemie (1983), p. 19] convient parfaitement.

Pour l'isolation des produits de réaction, on procède de la manière suivante: on détruit d'abord l'excès d'hydrure de potassium à l'aide d'isopropanol et le mélange résultant est versé dans de l'eau. La phase aqueuse contenant le sel alcalin est ensuite acidifiée et extraite à l'aide de solvants organiques. Les acides sont ainsi obtenus directement par évaporation de la phase organique. Suivant la variante B., la phase aqueuse est saturée avec du KCl afin de maintenir l'éther couronne dans la solution aqueuse même.

Nous avons observé que la réaction qui caractérise le procédé de la présente invention procède avec rétention complète de la configuration. C'est ainsi qu'en utilisant comme produit de départ un alcoolate optiquement actif de structure isomérique défini l'on obtient l'acide correspondant de même configuration isomérique.

L'invention est illustrée plus en détail dans les exemples qui suivent dans lesquels les températures sont indiquées en degrés centigrades et les abréviations ont le sens usuel.

Exemple 1

Acide 2,6-diméthyl-2-vinyl-5-hepténoïque

3,52 grammes de KH dispersés dans une huile minérale (Aldrich, 35% KH, 30,7 mM) ont été lavés à plusieurs reprises avec du pentane et l'huile est éliminée par décantation. Le KH qui en est résulté a été suspendu dans 30 ml de benzène anhydre. A la suspension résultante refroidie, on a ajouté en 5 minutes sous agitation 3 g (19 mM) de linalol dans 10 ml de benzène. L'addition s'accompagne de libération d'hydrogène et le mélange a été maintenu sous agitation pendant 2 h à température ambiante. Ces opérations ont été effectuées sous argon dans un récipient en verre dont la forme épouse celle de l'autoclave, le récipient ayant été fermé à l'aide d'une capsule en caoutchouc. Le tout a été ensuite introduit dans l'autoclave et le récipient a été ouvert, puis à température ambiante, on a introduit du CO (pureté 99,97%) jusqu'à l'obtention d'une pression de 360 bar. L'autoclave a été ensuite fermé et chauffé en 40 min. à 130°, la pression de CO atteint ainsi 430 bar, puis il a été agité à 130° pendant 15 h. Après refroidissement, on a éliminé l'excès de gaz et l'autoclave a été ouvert. Le contenu du récipient en verre a été traité avec quelques gouttes d'eau ou d'isopropanol afin d'éliminer l'excès de KH et le mélange qui en résulte a été versé dans de l'eau (50 ml) et de l'éther diéthylique. Le mélange a été ensuite extrait avec de l'éther.

La phase aqueuse alcaline restante a été acidifiée avec du HCl aqueux, saturée avec du NaCl et extraite avec de l'éther. Les extraits éthérés ainsi obtenus ont été séchés et concentrés pour fournir 2 g de l'acide désiré.

Exemple 2

Acide 2,6-diméthyl-2-vinyl-5-hepténoïque

L'alcoolate potassique du (S)-(+)-linalol a été préparé à partir d'un alcool ayant une pureté énantiomérique de 62 ± 5% (1,54 g) et 1,73 g de KH dispersé dans une huile minérale (voir Exemple 1 ci-dessus). La solution benzénique résultante a été traitée avec 4,75 g de 18-éther couronne-6 (18-crown-6; Aldrich, 18 mM) et le mélange obtenu a été agité pendant 1 h. Il en a résulté une solution qui a été introduite, comme indiqué à l'Exemple 1, dans un autoclave et du CO a été introduit jusqu'à l'obtention d'une pression de 50 bar. A cette pression, l'autoclave a été fermé, agité magnétiquement et chauffé à 40° pendant 113 h. La pression atteint ainsi 55 bar. Après refroidissement, le mélange de réaction a été traité comme indiqué à l'exemple précédent. La fraction acide a été distillée au moyen d'un four à boules (température bain 100-250°/0,66 Pa) pour fournir 0,471 g d'un mélange contenant 55,8% de l'acide désiré: $[\alpha]^{20}_{D}$ =+2,11±0,12° (c=0,760 g/100 ml $CHCl_3$).

Par estérification à l'aide d'une réaction avec du diazométhane, on a pu obtenir l'ester méthylique correspondent ayant $[\alpha]^{20}_{D}$=+2,05±0,08° (c=1,290 g/100 ml $CHCl_3$), pureté énantiomérique 55±5%. Le 2,6-diméthyl-2-vinyl-5-hepténoate de méthyle présente une note olfactive intéressante de type floral, frais.

Exemple 3

Acide 2-vinyl-2-pentyl-3-buténoïque

1,00 gramme de 3-vinyl-1-octène-3-ol (pureté 81%) a été transformé en l'alcoolate potassique correspondant comme indiqué à l'Exemple 1 et soumis à réaction avec du CO selon la même procédure que celle suivie pour le linalol (pression de CO: 45 bar; température: 120°, temps de réaction: 12 h). L'acide désiré a été obtenu sous forme d'un mélange dont la teneur en l'acide était d'environ 55%.

En effectuant la réaction en présence de 18-éther couronne-6, comme décrit à l'Exemple 2 (pression CO: 360 bar; température ambiante; temps de réaction: 65 h), on a pu obtenir l'acide désiré sous forme d'un mélange dont le contenu en l'acide était de 60,6%.

Par estérification au diazométhane, on a pu isoler l'ester méthylique correspondant, ou 2-vinyl-2-pentyl-3-buténoate de méthyle.

RMN (360 MHz): 0,88 (3H, t); 1,20-1,37 (6H, m); 2,76-2,85 (2H, m); 3,72 (3H, s); 5,12 (2H, d); 5,23 (2H, d); 6,04 (2H, dxd) delta ppm;

SM : $M^+$ = 196; m/e : 139 (28), 126 (27), 125 (23), 95 (36), 94 (26), 81 (67), 79 (34), 67 (100).

ANNEXE

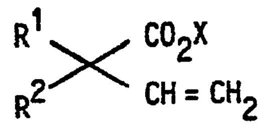

(I)

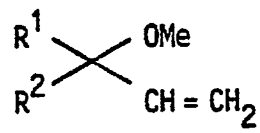

(II)

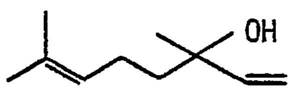

et

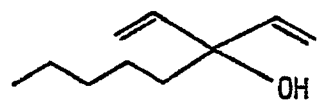

(III)

(IV)

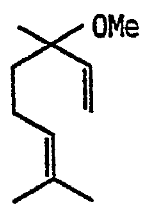

(V)

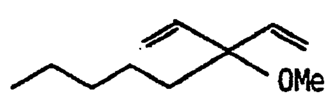

(VI)

## Revendications

1. Procédé pour la préparation d'acides carboxyliques ou de leurs sels alcalins de formule (I)

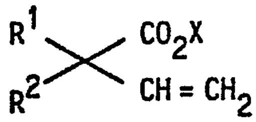

(I)

dans laquelle X définit un hydrogène, ou un atome de métal alcalin, et $R^1$ et $R^2$ identiques ou différents représentent chacun un reste alkyle linéaire ou ramifié, saturé ou insaturé, caractérisé en ce qu'on

A. traite un alcoolate de formule (II)

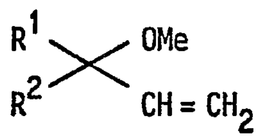

(II)

dans laquelle Me représente un atome de métal alcalin, en solution dans un solvant organique inerte, dans les conditions de la réaction, avec du CO à une pression comprise entre 35 et 450 bar et à une température comprise entre 120 et 160°C, traite ensuite avec de l'eau le carboxylate alcalin obtenu et acidifie enfin la solution aqueuse résultante pour fournir l'acide désiré, ou

B. traite avec du CO un alcoolate de formule (II) en solution dans un solvant organique inerte dans les conditions de la réaction en présence d'un réactif ligand des cations alcalins, à une pression comprise entre 40 et 80 bar et à une température de 15 à 45°C, traite ensuite avec de l'eau le carboxylate alcalin obtenu et acidifie enfin la solution aqueuse résultante pour fournir l'acide désiré.

2. Procédé suivant la revendication 1, caractérisé en ce qu'on utilise comme alcoolate de formule (II) le composé de formule (V)

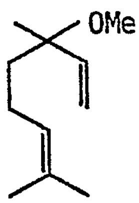

(V)

dans laquelle Me représente un atome de métal alcalin et qu'on obtient l'acide 2,6-diméthyl-2-vinyl-5-hepténoïque ou l'un de ses sels alcalins correspondants.

3. Procédé suivant la revendication 1, caractérisé en ce qu'on utilise comme alcoolate de formule (II) le composé de formule (VI)

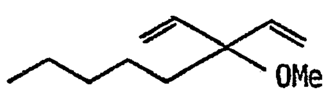

(VI)

dans laquelle Me représente un atome de métal alcalin et qu'on obtient l'acide 2-vinyl-2-pentyl-3-buténoïque ou l'un de ses sels alcalins correspondants.

4. Procédé suivant la revendication 1, caractérisé en ce qu'on utilise comme alcoolate de formule (II) un alcoolate potassique.

5. Procédé suivant la revendication 1, caractérisé en ce que le réactif ligand des cations alcalins est choisi parmi les éthers couronne.

6. Procédé suivant la revendication 5, caractérisé en ce que l'éther couronne est le 18-couronne-6.

## Patentansprüche

1. Verfahren zur Herstellung von Carbonsäuren oder von ihren alkalischen Salzen der Formel (I)

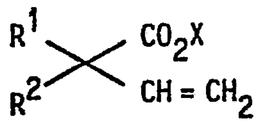

(I)

in welchem X ein Wasserstoffatom oder ein Alkalimetall bedeutet, und jeder der Reste $R^1$ und $R^2$, welche verschieden oder gleich sein können, einen linearen oder verzweigten, gesättigten oder ungesättigten Alkylrest bedeuten kann, dadurch gekennzeichnet, dass man:

A. ein Alkoholat der Formel (II)

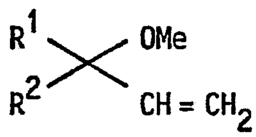

(II)

in welchem Me ein Alkalimetallatom darstellt, in Lösung in einem inerten organischen Lösungsmittel unter den Reaktionsbedingungen mit CO bei einem Druck zwischen 35 und 450 Bar und einer Temperatur zwischen 120 und 160°C behandelt, danach das so erhaltene Alkalicarboxylat mit Wasser behandelt und endlich die resultierende wässerige Lösung ansäuert um die gewünschte Säure zu erhalten;

B. ein Alkoholat der Formel (II), in Lösung in einem inerten organischen Lösungsmittel unter den Reaktionsbedingungen in Gegenwart einer Alkalimetallkationen komplexierenden Verbindung mit CO bei einem Druck zwischen 40 und 80 Bar und einer Temperatur zwischen 15 und 45°C behandelt, danach das so erhaltene Alkalicarboxylat mit Wasser behandelt und endlich die resultierende wässerige Lösung ansäuert um die gewünschte Säure zu erhalten;

2. Verfahren gemäss Anspruch 1, dadurch gekennzeichnet, dass man, als Alkoholat der Formel (II), die Verbindung der Formel (V)

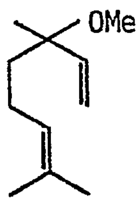

(V)

in welchem Me ein Alkalimetall bedeutet, verwendet, und dass man die 2,6-Dimethyl-2-vinyil-5-heptensäure, oder eines der ihr entsprechenden alkalischen Salze, erhält.

3. Verfahren gemäss Anspruch 1, dadurch gekennzeichnet, dass man, als Alkoholat der Formel (II), die Verbindung der Formel (VI)

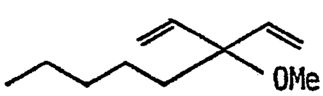

(VI)

in welchem Me ein Alkalimetall bedeutet, verwendet, und dass man die 2-Vinyl-2-pentyl-3-butensäure, oder eines der ihr entsprechenden alkalischen Salze erhält.

4. Verfahren gemäss Anspruch 1, dadurch gekennzeichnet dass man, als Alkoholat der Formel (II), ein Kaliumalkoholat verwendet.

5. Verfahren gemäss Anspruch 1, dadurch gekennzeichnet, dass die Alkalimetallkationen komplexierende Verbindung aus der Reihe der Kronenäther ausgewählt wird.

6. Verfahren gemäss Anspruch 1, dadurch gekennzeichnet, dass der Kronenäther die 18-Krone-6 ist.

## Claims

1. Process for the preparation of carboxylic acids or alkali salt derivatives thereof of formula (I)

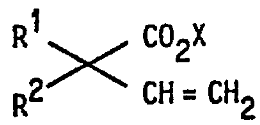

(I)

wherein X stands for a hydrogen or an alkali metal atom, and $R^1$ and $R^2$ are identical or different and represent each a linear or branched, saturated or unsaturated alkyl radical, characterized in that:

A. an alkoxide formula (II)

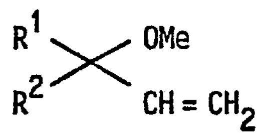

(II)

wherein Me stands for an alkali metal atom, in solution in an inert organic solvent under the reaction conditions, is treated with CO at a pressure of between 35 and 450 bar and at a temperature of between 120 and 160°C, the thus obtained alkaline carboxylate is then treated with water and the resulting aqueous solution is finally acidified to give the desired acid, or

B. an alkoxide of formula (II), in solution in an inert organic solvent under the reaction conditions, in the presence of a ligand of alkali metal cations, is treated with CO at a pressure of between 40 and 80 bar and at a temperature of 15 to 45°C, the thus obtained alkali carboxylate is then treated with water and the resulting aqueous solution is finally acidified to give the desired acid.

2. Process according to claim 1, characterized in that one uses as alkoxide of formula (II) the compound of formula (V)

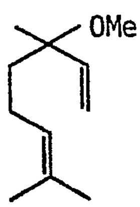

(V)

wherein Me stands for an alkali metal atom, and in that 2,6-dimethyl-2-vinyl-5-heptenoic acid or one of its corresponding alkali metal salts is obtained.

3. Process according to claim 1, characterized in that one uses as alkoxide of formula (II) the compound of formula (VI)

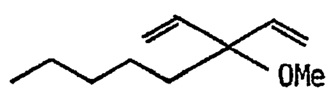

(VI)

wherein Me represents an alkali metal atom, and in that 2-vinyl-2-pentyl-3-butenoic acid or one of its corresponding alkali metal salts is obtained.

4. Process according to claim 1, characterized in that a potassium alkoxide is used as alkoxide of formula (II).

5. Process according to claim 1, characterized in that the ligand of alkali metal cations is chosen amongst the crown ethers.

6. Process according to claim 5, characterized in that the crown ether is the 18-crown-6.